# EUROPEAN PATENT APPLICATION

(11) **EP 4 286 375 A1**
(43) Date of publication of application: **06.12.2023**
(21) Application number: 21923212.1
(22) Date of filing: 20.12.2021
(51) Int. Cl.: C07D 309/04, C07D 333/20, B01D 53/14, C07D 295/13, C01B 32/50, C07D 307/14, C07D 307/52

(54) **CARBON DIOXIDE ABSORBER**

(30) Priority: 27.01.2021 JP 2021011420
(71) Applicant: MITSUBISHI GAS CHEMICAL COMPANY, INC., Chiyoda-ku Tokyo 100-8324 (JP)
(72) Inventor: KOUNO, Kazuki, Hiratsuka-shi, Kanagawa 254-0016 (JP); KAWASHIMA, Yuki, Hiratsuka-shi, Kanagawa 254-0016 (JP); ASAI, Ryo, Tokyo 125-8601 (JP); KIRINO, Tomoaki, Tokyo 100-8324 (JP)
(74) Representative: Hoffmann Eitle
(86) International application number: PCT/JP2021/046959
(87) International publication number: WO 2022/163209

(57) **Abstract**

A carbon dioxide absorbent containing an amine compound (A) having a heterocyclic structure (X) selected from the group consisting of an oxygen-containing heterocyclic structure and a sulfur-containing heterocyclic structure, a content of the amine compound (A) being 65% by mass or more.

## Description

### Technical Field

The present invention relates to a carbon dioxide absorbent.

### Background Art

From a viewpoint of global warming issues, there is a need to reduce carbon dioxide.

One of methods for reducing carbon dioxide is technology that effectively captures a high concentration (about 10 to 30% by volume) of carbon dioxide from exhaust gases exhausted from a thermal power plant and so on, and stores it by burying into the ground and the sea (CCS: Carbon dioxide Capture and Storage). The technology regarding carbon dioxide absorbents used in CCS includes, for example, those described in Patent Literatures 1 to 4.

In Patent Literature 1, described is a method for capturing carbon dioxide by using specific alkanolamines as the carbon dioxide absorbent.

In Patent Literature 2, described is that a carbon dioxide absorption liquid containing carbon dioxide chemoabsorbent amines having a nitrogen-hydrogen bond and a tertiary amine solvent not having the nitrogen-hydrogen bond is used as the carbon dioxide absorbent.

In Patent Literature 3, described is an absorption liquid for acidic gaseous compounds that exhibits CO₂ absorption capability, the absorption liquid comprising a tertiary alkanolamine component composed of one or more tertiary alkanolamines and an active agent for CO₂ absorption by the tertiary alkanolamine component, and the above active agent being constituted by an amino compound having a specific structure.

In Patent Literature 4, described is an aqueous composition for capturing carbon dioxide from a high-pressure gas containing carbon dioxide, the aqueous composition comprising 10 to 60% by mass of a heterocyclic compound, the heterocyclic compound being at least one selected from the group consisting of (i) a 5 to 14 membered saturated heterocyclic ring having 1 to 4 nitrogen atoms and optionally having 1 to 10 oxygen atoms and (ii) a 5 to 14 membered unsaturated heterocyclic ring having 1 to 4 nitrogen atoms.

In recent years, the technology of directly capturing carbon dioxide at a low concentration (about 0.04% by volume) from the air (DAC: Direct Air Capture) has gained attention. The technology with respect to the carbon dioxide absorbent used in DAC includes, for example, one described in Patent Literature 5.

In Patent Literature 5, described is an absorbent of carbon dioxide from the air, containing an alkylamine substituted by a hydroxy group or an amino group which may be substituted.

### Citation List

### Patent Literature

PTL1: JP 2008-13400 A
PTL2: JP 2017-104776 A
PTL3: JP 02-504367 A
PTL4: JP 2011-25100 A
PTL5: JP 2017-031046 A

### Summary of Invention

### Technical Problem

According to the present inventors' studies, it has become apparent that there is a room for improvement for the carbon dioxide absorbent described in Patent Literature 5 used in DAC, in terms of carbon dioxide desorption performance.

The present invention was made in view of the above circumstances, and provides a carbon dioxide absorbent having a good absorption of carbon dioxide from the air, as well as an excellent carbon dioxide desorption performance. Solution to Problem

The present inventors made intensive studies in order to solve the above problem. As a result, they have found that a carbon dioxide absorbent comprising a specific amount of an amine compound (A) having a heterocyclic structure (X) selected from the group consisting of an oxygen-containing heterocyclic structure and a sulfur-containing heterocyclic structure has a good absorption of carbon dioxide from the air, as well as an excellent carbon dioxide desorption performance, and thus have completed the present invention.

That is, according to the present invention, the following carbon dioxide absorbent is provided.

[1] A carbon dioxide absorbentcomprising an amine compound (A) having a heterocyclic structure (X) selected from the group consisting of an oxygen-containing heterocyclic structure and a sulfur-containing heterocyclic structure, a content of the amine compound (A) being 65% by mass or more.
[2] The carbon dioxide absorbent according to the above [1], wherein a maximum carbon dioxide release temperature of the amine compound (A) is 120°C or less measured by the following method:
   heating the amine compound (A) with carbon dioxide absorbed from 23°C to 250°C at 10°C/minute of a heating rate, measuring a temperature at which an endothermic amount associated with desorption of carbon dioxide reaches a maximum, and letting the temperature be the maximum carbon dioxide release temperature.
[3] The carbon dioxide absorbent according to the above [1] or [2], wherein an acid dissociation constant (pKa) of the amine compound (A) is 8.0 or more and 12.0 or less.
[4] The carbon dioxide absorbent according to any one of the above [1] to [3], wherein a molecular weight of the amine compound (A) is 90 or more and 1,000 or less.
[5] The carbon dioxide absorbent according to any one of the above [1] to [4], wherein a maximum endothermic temperature of the amine compound (A) is 120°C or more and 300°C or less measured by the following method:
   heating the amine compound (A) from 23°C to 350°C at a 10°C/minute of a heating rate, measuring a temperature at which an endothermic amount associated with volatilization of the amine compound (A) reaches a maximum, and letting the temperature be the maximum endothermic temperature of the amine compound (A).
[6] The carbon dioxide absorbent according to any one of the above [1] to [5], wherein an amine value of the amine compound (A) is 400 mgKOH/g or more and 1,500 mgKOH/g or less.
[7] The carbon dioxide absorbent according to any one of the above [1] to [6], wherein amino groups of the amine compound (A) is 1 or more and 4 or less.
[8] The carbon dioxide absorbent according to any one of the above [1] to [7], wherein the heterocyclic structure (X) comprises at least one selected from the group consisting of a 5 membered ring and a 6-membered ring.
[9] The carbon dioxide absorbent according to any one of the above [1] to [8], wherein the amine compound (A) comprises at least one selected from the group consisting of 2,5-bisaminomethylfuran and derivatives thereof, 2,5-bis(aminomethyl)tetrahydrofuran and derivatives thereof, furfurylamine and derivatives thereof, tetrahydrofurfurylamine and derivatives thereof, 4-aminomethyltetrahydropyran and derivatives thereof, 4-(2-aminoethyl)morpholine and derivatives thereof, and 2-thiophenemethylamine and derivatives thereof.
[10] The carbon dioxide absorbent according to any one of the above [1] to [9], wherein a content of water is 30% by mass or less.
[11] The carbon dioxide absorbent according to any one of the above [1] to [10], which is used for directly absorbing carbon dioxide from the air.

### Advantageous Effects of Invention

According to the present invention, a carbon dioxide absorbent having a good absorption of carbon dioxide from the air as well as an excellent carbon dioxide desorption performance can be provided.

### Description of Embodiments

Embodiments of the present invention (hereinafter, simply referred to as "the present embodiments") will be described in detail. The following embodiments are examples to explain the present invention and do not limit the contents of the present invention. The present invention can be implemented with appropriate modifications within the scope of the invention. In the present embodiments, it is considered that provisions that are preferred can be adopted arbitrarily, and combinations of preferred ones are more preferred. In the present embodiments, description of "XX to YY" means "XX or more and YY or less".

A carbon dioxide absorbent of the present embodiments comprises an amine compound (A) having a heterocyclic structure (X) selected from the group consisting of an oxygen-containing heterocyclic structure and a sulfur-containing heterocyclic structure, and a content of the amine compound (A) is 65% by mass or more.

Here, in the present embodiments, the oxygen-containing heterocyclic structure or the sulfur-containing heterocyclic structure refers to the heterocyclic structure that contains an oxygen atom or a sulfur atom as a heteroatom in a cyclic structure.

As the oxygen-containing heterocyclic structure with respect to the present embodiments, preferable are the heterocyclic structure containing a nitrogen atom and the oxygen atom as the heteroatoms in the cyclic structure and the heterocyclic structure containing only the oxygen atom as the heteroatom in the cyclic structure, more preferable are the heterocyclic structure containing only one oxygen atom as the heteroatom in the cyclic structure and the heterocyclic structure containing only one oxygen atom and only one nitrogen atom as the heteroatoms in the cyclic structure, even more preferable is the heterocyclic structure containing only one oxygen atom as the heteroatom in the cyclic structure.

Further, as the sulfur-containing heterocyclic structure with respect to the present embodiments, preferable are the heterocyclic structure containing the nitrogen atom and the sulfur atom as the heteroatoms in the cyclic structure and the heterocyclic structure containing only the sulfur atom as the heteroatom in the cyclic structure, more preferable are the heterocyclic structure containing only one sulfur atom as the heteroatom in the cyclic structure and the heterocyclic structure containing only one sulfur atom and only one nitrogen atom as the heteroatoms in the cyclic structure, even more preferable is the heterocyclic structure containing only one sulfur atom as the heteroatom in the cyclic structure.

Also, the amine compound (A) having the heterocyclic structure (X) may be any of a cis-isomer, a trans-isomer or a mixture of the cis-isomer and the trans-isomer.

The carbon dioxide absorbent of the present embodiments comprises a specific amount of the amine compound (A) and is an absorbent having a good absorption of carbon dioxide from the air, as well as an excellent carbon dioxide desorption performance.

"Good absorption of carbon dioxide from the air" in the present embodiments means that the absorbed amount of carbon dioxide at a low concentration (about 0.04% by volume) from the air is large, and more specifically, in "Evaluation of Absorbed Amount of Carbon Dioxide from Air" in Examples described later, a change of carbon dioxide concentration in a desiccator after 24 hours is 150 ppm or more, preferably 180 ppm or more, more preferably 230 ppm or more, even more preferably 280 ppm or more. In addition, "excellent carbon dioxide desorption performance " in the present embodiments means that the carbon dioxide absorbed is easily desorbed at a lower temperature, and the lower the maximum carbon dioxide release temperature is, the more excellent the carbon dioxide desorption performance is.

The carbon dioxide absorbent of the present embodiments comprises the specific amount of the amine compound (A) having the heterocyclic structure (X) selected from the group consisting of the oxygen-containing heterocyclic structure and the sulfur-containing heterocyclic structure. By comprising the specific amount of the amine compound (A), a good absorption of carbon dioxide from the air, as well as an excellent carbon dioxide desorption performance can be obtained. The reason therefor is not certain, but is considered to be as follows.

First, the amine compound (A) having the heterocyclic structure (X) is considered to absorb a large amount of carbon dioxide because of its strong basicity.

Further, the amine compound (A) having the heterocyclic structure (X) is considered to be excellent in carbon dioxide release performance because it has a structure with a large steric hindrance, as well as the adsorption between the amino group and the carbon dioxide is weakened by the oxygen atom or the sulfur atom.

For the above reasons, it is considered that the carbon dioxide absorbent of the present embodiments has a good absorption of carbon dioxide from the air, as well as an excellent carbon dioxide desorption performance.

The carbon dioxide absorbent of the present embodiments can be suitably used for the technology of directly absorbing carbon dioxide (DAC) from the air because it has a good absorption of carbon dioxide from the air.

In addition, the carbon dioxide absorbent of the present embodiments can be suitably used, for example, in case of capturing carbon dioxide at a low concentration of 0.01% by volume or more and 1% by volume or less.

The heterocyclic structure (X) of the amine compound (A) preferably comprises at least one selected from the group consisting of a 5-membered ring and a 6-membered ring from the viewpoint of improving absorption of carbon dioxide from the air and carbon dioxide desorption.

Further, it is preferable that the amine compound (A) has one heterocyclic structure (X). Namely, it is preferable that the amine compound (A) is a monocyclic compound.

The number of amino groups in the amine compound (A) is preferably 1 or more and 4 or less, more preferably 1 or more and 3 or less, even more preferably 1 or 2, still more preferably 2, from the viewpoint of improving absorption of carbon dioxide from the air and carbon dioxide desorption.

As the amino groups, an amino group having a nitrogen-hydrogen bond is preferable and a primary amino group is more preferable, from the viewpoint of further improving the amount of carbon dioxide absorbed from the air.

From the viewpoint of further improving absorption of carbon dioxide from the air and carbon dioxide desorption, the amine compound (A) preferably comprises at least one selected from the group consisting of 2,5-bisaminomethylfuran and derivatives thereof, 2,5-bis(aminomethyl)tetrahydrofuran and derivatives thereof, furfurylamine and derivatives thereof, tetrahydrofurfurylamine and derivatives thereof, 4-aminomethyltetrahydropyran and derivatives thereof, 4-(2-aminoethyl)morpholine and derivatives thereof, and 2 -thiophenemethylamine and derivatives thereof, more preferably comprises at least one selected from the group consisting of the 2,5-bisaminomethylfuran and derivatives thereof, the 2,5-bis(aminomethyl)tetrahydrofuran and derivatives thereof, the 4-aminomethyltetrahydropyran and derivatives thereof, and the 4-(2-aminoethyl)morpholine and derivatives thereof, even more preferably comprises at least one selected from the group consisting of the 2,5-bisaminomethylfuran and derivatives thereof, the 2,5-bis(aminomethyl)tetrahydrofuran and derivatives thereof, and the 4-(2-aminoethyl)morpholine and derivatives thereof, and still more preferably comprises at least one selected from the group consisting of the 2,5-bisaminomethylfuran, the 2,5-bis(aminomethyl)tetrahydrofuran, and the 4-(2-aminoethyl)morpholine.

Here, the derivatives of the above various amines include, for example, a compound in which at least one of the hydrogen atoms in the amino group is substituted by a hydrocarbon group having 1 or more and 10 or less carbon atoms, and optionally having at least one substituent selected from the group consisting of the amino group, a cyano croup and a phenyl group, preferably an alkyl group having 1 or more and 4 or less carbon atoms, and optionally having at least one substituent selected from the group consisting of the amino group, the cyano group and the phenyl group, more preferably the alkyl group having 1 or more and 4 or less carbon atoms, and optionally having at least one substituent selected from the group consisting of the amino group and the cyano group, even more preferably the alkyl group having 2 or more and 4 or less carbon atoms, and optionally having at least one substituent selected from the group consisting of the amino group and the cyano group.

Also, the derivatives of the above various amines include, for example, a compound in which at least some of the hydrogen atoms of the heterocyclic structure are substituted by the hydrocarbon group having 1 or more and 4 or less carbon atoms, preferably the alkyl group having 1 or more and 3 or less carbon atoms, more preferably a methyl group or an ethyl group, even more preferably the methyl group.

Also, from the viewpoint of further improving absorption of carbon dioxide from the air and carbon dioxide desorption, the amine compounds (A) preferably comprises at least one selected from the group consisting of the 2,5-bisaminomethylfuran, the 2,5-bis(aminomethyl)tetrahydrofuran, the furfurylamine, 5-methylfurfurylamine, the 4-aminomethyltetrahydropyran, the 4-(2-aminoethyl)morpholine and the 2-thiophenemethylamine, more preferably comprises at least one selected from the group consisting of the 2,5-bisaminomethylfuran, the 2,5-bis(aminomethyl)tetrahydrofuran, the 4-aminomethyltetrahydropyran, and the 4-(2-aminoethyl)morpholine, and even more preferably comprises at least one selected from the group consisting of the 2,5-bisaminomethylfuran, the 2,5-bis(aminomethyl)tetrahydrofuran, and the 4-(2- aminoethyl)morpholine.

These amine compounds (A) can be used alone or in combination of two or more.

The content of the amine compound (A) in the carbon dioxide absorbent of the present embodiments is, from the viewpoint of improving the absorbed amount of carbon dioxide from the air and the carbon dioxide desorption, 65% by mass or more, preferably 70% by mass or more, more preferably 80% by mass or more, even more preferably 90% by mass or more, still more preferably 95% by mass or more, yet more preferably 98% by mass or more, and preferably 100% by mass or less, when the total amount of the carbon dioxide absorbent is 100% by mass.

Further, the content of the amine compound (A) in the carbon dioxide absorbent of the present embodiments is, from the viewpoint of improving absorption of carbon dioxide from the air and carbon dioxide desorption, preferably 65 parts by mass or more, more preferably 70 parts by mass or more, even more preferably 80 parts by mass or more, still more preferably 90 parts by mass or more, yet more preferably 95 parts by mass or more, even much more preferably 98 parts by mass or more, and preferably 100 parts by mass or less, when the total amount of amine compounds contained in the carbon dioxide absorbent is 100 parts by mass.

A content of water in the carbon dioxide absorbent of the present embodiments is, from the viewpoint of improving absorption of carbon dioxide from the air and carbon dioxide desorption, preferably 30% by mass or less, more preferably 15% by mass or less, even more preferably 10% by mass or less, still more preferably 5% by mass or less, yet more preferably 1% by mass or less, even much more preferably 0.5% by mass or less, still much more preferably 0.1% by mass or less, yet much more preferably 0.01% by mass or less, and it is even still much more preferable that the carbon dioxide absorbent of the present embodiments is substantially free of water. Here, "substantially free of water" means that water is not intentionally added and does not exclude the presence of a small amount of water as an impurity.

The maximum carbon dioxide release temperature of the amine compound (A) as measured by the following method is, from the viewpoint of improving the carbon dioxide release performance, preferably 120°C or less, more preferably 110°C or less, even more preferably 100°C or less, still more preferably 90°C or less, yet more preferably 88°C or less. A lower limit of the above maximum carbon dioxide release temperature is not limited, but is, e.g., 40°C or more, and from the viewpoint of further improving the absorbed amount of carbon dioxide from the air, preferably 70°C or more, more preferably 75°C or more.

### (Method)

The amine compound (A) is heated with carbon dioxide absorbed from 23°C to 250°C at 10°C/minute of a heating rate, and a temperature at which an endothermic amount associated with desorption of carbon dioxide reaches a maximum is measured, and this temperature is taken as the maximum carbon dioxide release temperature. Here, the amine compound (A) with carbon dioxide absorbed can be prepared, for example, by allowing 5 mmol of the amine compound (A) to stand in the air at 23°C and 50% RH for 24 hours.

An acid dissociation constant (pKa) of the amine compound (A) is, from the viewpoint of improving the absorbed amount of carbon dioxide from the air, preferably 8.0 or more, more preferably 8.5 or more, and even more preferably 8.8 or more, and from the viewpoint of improving the carbon dioxide release performance, preferably 12.0 or less, more preferably 11.0 or less, and even more preferably 10.5 or less.

In the present embodiments, the acid dissociation constant of the amine compound (A) is a value obtained by the following measurement method based on an acid-base titration method.
(1) Dissolving 0.2 g of the amine compound (A) in 30 mL of purified water.
(2) Calculating the acid dissociation constant (pKa) by titrating the solution obtained in the above (1) with 0.1 N perchloric acid-acetic acid solution using an automatic potentiometric titrator (for example, AT-610 produced by Kyoto Electronics Manufacturing Co., Ltd.).

Still, let a temperature at a time of measurement be 25±2°C.

The molecular weight of the amine compound (A) is, from the viewpoints of suppressing weight loss during in heat treatment to release carbon oxide, and further improving repeated usability, and from the viewpoint of further improving the absorbed amount of carbon dioxide from the air, preferably 90 or more, more preferably 100 or more, even more preferably 110 or more, still more preferably 115 or more, yet more preferably 120 or more, and from the viewpoint of improving the absorbed amount of carbon dioxide from the air, preferably 1,000 or less, more preferably 500 or less, even more preferably 300 or less, still more preferably 200 or less, yet more preferably 150 or less.

A maximum endothermic temperature of the amine compound (A) as measured by the following method is, from the viewpoints of suppressing weight loss in heat treatment to release carbon dioxide and further improving the repeated usability, and from the viewpoint of further improving the absorbed amount of carbon dioxide from the air, preferably 120°C or more, more preferably 130°C or more, even more preferably 140°C or more, still more preferably 143°C or more, yet more preferably 150°C or more, and from the viewpoint of further improving the absorbed amount of carbon dioxide from the air, preferably 300°C or less, more preferably 250°C or less, even more preferably 200°C or less, still more preferably 180°C or less, yet more preferably 160°C or less.

### (Method)

Heating the amine compound (A) from 23°C to 350°C at 10°C/minute of the heating rate, measuring a temperature at which an endothermic amount associated with the volatilization of the amine compound (A) reaches a maximum, and letting this temperature be the maximum endothermic temperature of the amine compound (A).

An amine value of the amine compound (A) is, from the viewpoint of further improving the absorbed amount of carbon dioxide from the air and the carbon dioxide desorption performance, preferably 400 mgKOH/g or more, more preferably 500 mgKOH/g or more, even more preferably 600 mgKOH/g or more, still more preferably 700 mgKOH/g or more, yet more preferably 800 mgKOH/g or more, and preferably 1,500 mgKOH/g or less, more preferably 1,200 mgKOH/g or less, even more preferably 1,000 mgKOH/g or less, still more preferably 900 mgKOH/g or less. The amine value indicates an amount of the amine in the compound and is number of mg of potassium hydroxide (KOH) of an equivalent amount to acid required to neutralize 1 g amount of the compound. The amine value can be measured by the following method in accordance with JIS K7237-1995.
(1) Dissolving 0.1 g of the amine compound (A) in 20 mL of acetic acid.
(2) Calculating the amine value by titrating the solution obtained in the above (1) with the 0.1 N perchloric acid-acetic acid solution using the automatic potentiometric titrator (for example, AT-610 produced by Kyoto Electronics Manufacturing Co., Ltd.).

The carbon dioxide absorbent of the present embodiments may comprise a component other than the amine compound (A), as appropriate, to the extent that the effect of the invention is not impaired. Examples of components other than the amine compound (A) includes a compound other than the amine compound (A) that can absorb carbon dioxide (e.g., methanol, polyethylene glycol or the like), water, an organic solvent, a degradation inhibitor, a defoaming agent, a viscosity adjuster, an antioxidant, and a desiccant for removing moisture (magnesium sulfate, molecular sieves or the like).

The organic solvent includes, for example, alcohol, dimethylacetamide, N-methylpyrrolidone, and dimethylformamide.

### Examples

Hereinafter, the present invention will be described with reference to examples, but the present invention is not limited to the scope of the examples. In the present examples, various measurements and evaluations were performed by the following methods.

### (Acid Dissociation Constant (pKa) of Amine Compound)

The acid dissociation constant of the amine compound was determined by the following method.
(1) Dissolving 0.2 g of the amine compound in 30 mL of the purified water.
(2) Calculating the acid dissociation constant (pKa) by titrating the solution obtained in the above (1) with the 0.1 N perchloric acid-acetic acid solution using the automatic potentiometric titrator (AT-610 produced by Kyoto Electronics Manufacturing Co., Ltd.).

Still, let the temperature at the time of measurement be 25±2°C.

### (Amine Value of Amine Compound)

The amine value was measured by the following measurement method in accordance with JIS K7237-1995.
(1) Dissolving 0.1 g of the amine compound in 20 mL of the acetic acid.
(2) Calculating the amine value by titrating the solution obtained in the above (1) with the 0.1 N perchloric acid-acetic acid solution using the automatic potentiometric titrator (AT-610 produced by Kyoto Electronics Manufacturing Co., Ltd.).

### (Maximum Endothermic Temperature of Amine Compound)

DSC measurements were performed on the amine compounds used in Examples and Comparative Examples to measure the maximum endothermic temperature of the amine compounds as follows. First, a differential scanning calorimetry was performed on the amine compounds using a differential thermogravimetric analyzer (Product Name: DTG-60 produced by Shimadzu Corporation) under conditions of 23 to 350°C of a measurement temperature range, 10°C/min of a heating rate, and a nitrogen atmosphere. From the DSC curve obtained thereby, the temperature at which the endothermic amount associated with the volatilization of the amine compound reached the maximum was calculated, and the temperature thereof was used as the maximum endothermic temperature of the amine compound.

### (Evaluation of Absorbed Amount of Carbon Dioxide from Air)

A carbon dioxide concentration meter and a petri dish were placed inside an openable desiccator (Internal Dimension: 370 mm x 260 mm x 272 mm). The amine compound (5 mmol) was then added to the petri dish in the desiccator, a door was immediately closed, and the carbon dioxide concentration in the desiccator was measured over time for 24 hours under an air environment of 23°C and 50% RH. The initial concentration of carbon dioxide was adjusted to about 400 ppm. Changes of the carbon dioxide concentration in the desiccator 24 hours after the amine compound was placed in the desiccator are shown in Table 1. Here, it is meant that the greater the change of the carbon dioxide concentration in the desiccator is, the greater the amount of the carbon dioxide absorbed by the carbon dioxide absorbent is.

### (Maximum Carbon Dioxide (CO₂) Release Temperature of Amine Compound)

After the above evaluation of the absorbed amount of carbon dioxide from the air was completed, the amine compound was removed from the desiccator as the amine compound having carbon dioxide absorbed. The DSC measurement was performed on the amine compound with carbon dioxide absorbed as follows, to measure the maximum carbon dioxide release temperature of the amine compound. First, the differential scanning calorimetry was performed on the amine compound using the differential thermogravimetric analyzer (Product Name: DTG-60 produced by Shimadzu Corporation) under the conditions of 23 to 250°C of a measurement temperature range, 10°C/min of the heating rate, and the nitrogen atmosphere. From the DSC curve obtained thereby, the temperature at which the endothermic amount associated with the desorption of carbon dioxide reached the maximum was calculated, and the temperature thereof was used as the maximum carbon dioxide release temperature of the amine compound.

In Examples and Comparative Examples, the following amine compounds were used.

### (Amine Compounds)

2,5-BATF: 2,5-bis(aminomethyl)tetrahydrofuran (prepared according to the following Synthesis Example 1)
2,5-BAF: 2,5-bisaminomethylfuran (produced by Carbosynth Limited)
4-aminomethyltetrahydropyran (produced by Tokyo Chemical Industry Co., Ltd.)
4-(2-aminoethyl)morpholine (produced by FUJIFILM Wako Pure Chemical Corporation)
2-thiophenemethylamine (produced by FUJIFILM Wako Pure Chemical Corporation)
Furfurylamine (produced by FUJIFILM Wako Pure Chemical Corporation)
5-methylfurfurylamine (produced by FUJIFILM Wako Pure Chemical Corporation)
MXDA: Meta-xylylenediamine (produced by Mitsubishi Gas Chemical Company, Inc.)
DETA: Diethylenetriamine (produced by Tokyo Chemical Industry Co., Ltd.)

### (Synthesis Example 1: Preparation of 2,5-BATF)

In a pressure-resistant autoclave, 20 g of the 2,5-bis(aminomethyl)furan, 5% by mass of Rh/C (8 g) as a catalyst, and 120 mL of THF as a solvent were charged. A hydrogen pressure in the pressure-resistant autoclave was then increased to 6 MPaG, and reaction was carried out while a temperature was maintained at 90°C for 1 hour, followed by cooling the pressure-resistant autoclave with ice water to stop the reaction. The catalyst was removed by filtrating the catalyst and a reaction liquid under a flow of an argon gas to obtain a filtrate containing a product. The filtrate containing the product was then concentrated and purified by reduced pressure distillation at a temperature of 120°C and pressure of 1 mbar, to obtain 2,5-bis(aminomethyl)tetrahydrofuran.

In addition, the 2,5-bis(aminomethyl)furan produced by Carbosynth Limited was used. The THF used was Wako special grade tetrahydrofuran (without stabilizers) produced by FUJIFILM Wako Pure Chemical Corporation. The Rh/C which was a rhodium catalyst supported on carbon, and produced by N.E. Chemcat Corporation, was used.

### (Examples 1 to 7 and Comparative Examples 1 to 2)

In Examples 1 to 7 and Comparative Examples 1 to 2, each of the above evaluations was performed using the carbon dioxide absorbent in which the content of the amine compound shown in Table 1 was 100% by mass. The results obtained are shown in Table 1.

**Table 1**

| | Amine Compounds | | | | | | | Evaluation of CO₂ Absorbed Amount |
|---|---|---|---|---|---|---|---|---|
| | Name | Structural Formula | Molecular Weight [-] | Maximum CO₂ Release Temperature [°C] | Maximum Endothermic Temperature [°C] | Amine Value [mgKOH/g] | pKa | Changes of CO₂ Concentration [ppm] |
| | | | | | | | | 24h |
| Example 1 | 2,5-BATF | 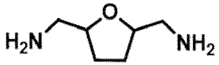 | 130.2 | 80.3 | 155.9 | 862 | 9.4 | 300 |
| Example 2 | 2,5-BAF | 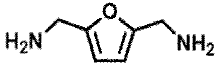 | 126.2 | 85.0 | 154.2 | 890 | 9.0 | 306 |
| Example 3 | 4-aminomethylte trahydropyran | 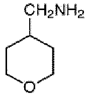 | 115.2 | 111.0 | 127.4 | 487 | 10.0 | 389 |
| Example 4 | 4-(2-aminoethyl)mor pholine | 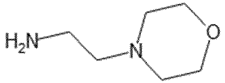 | 130.2 | 81.5 | 145.0 | 862 | 10.1 | 400 |
| Example 5 | 2-thiophenemeth ylamine | 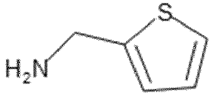 | 113.2 | 83.6 | 140.1 | 496 | 9.5 | 249 |
| Example 6 | Furfurylamine | 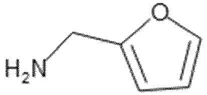 | 97.1 | 66.7 | 92.4 | 578 | 9.2 | 188 |
| Example 7 | 5-methylfurfuryl amine | 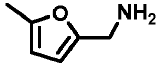 | 111.1 | 80.7 | 115.3 | 505 | 9.2 | 226 |
| Comparative Example 1 | MXDA | 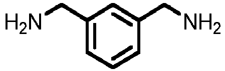 | 136.2 | 135.5 | 183.5 | 824 | 9.5 | 329 |
| Comparative Example 2 | DETA | 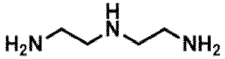 | 103.2 | 127.8 | 127.8 | 1632 | 9.7 | 300 |

From Table 1, it can be seen that the carbon dioxide absorbents in Examples containing the specific amount of the amine compound (A) having the heterocyclic structure (X) selected from the group consisting of the oxygen-containing heterocyclic structure and the sulfur-containing heterocyclic structure had a good range of the absorbed amount of carbon dioxide from the air and further a low maximum carbon dioxide release temperature. That is, it can be seen that the carbon dioxide absorbent of the present invention has a good absorption of carbon dioxide from the air, as well as an excellent carbon dioxide desorption performance.

## Claims

1. A carbon dioxide absorbent comprising an amine compound (A) having a heterocyclic structure (X) selected from the group consisting of an oxygen-containing heterocyclic structure and a sulfur-containing heterocyclic structure, a content of the amine compound (A) being 65% by mass or more.

2. The carbon dioxide absorbent according to claim 1, wherein a maximum carbon dioxide release temperature of the amine compound (A) is 120°C or less as measured by the following method,
the method comprising:
heating the amine compound (A) having carbon dioxide absorbed from 23°C to 250°C at a heating rate of 10°C/minute;
measuring a temperature at which an endothermic amount associated with desorption of the carbon dioxide reaches a maximum; and
taking the temperature as the maximum carbon dioxide release temperature.

3. The carbon dioxide absorbent according to claim 1 or 2, wherein an acid dissociation constant (pKa) of the amine compound (A) is 8.0 or more and 12.0 or less.

4. The carbon dioxide absorbent according to any one of claims 1 to 3, wherein a molecular weight of the amine compound (A) is 90 or more and 1,000 or less.

5. The carbon dioxide absorbent according to any one of claims 1 to 4, wherein a maximum endothermic temperature of the amine compound (A) is 120°C or more and 300°C or less as measured by the following method,
the method comprising:
heating the amine compound (A) from 23°C to 350°C at a heating rate of 10°C/minute,
measuring a temperature at which an endothermic amount associated with volatilization of the amine compound (A) reaches a maximum; and
taking the temperature as a maximum endothermic temperature of the amine compound (A).

6. The carbon dioxide absorbent according to any one of claims 1 to 5, wherein an amine value of the amine compound (A) is 400 mgKOH/g or more and 1,500 mgKOH/g or less.

7. The carbon dioxide absorbent according to any one of claims 1 to 6, wherein the number of amino groups of the amine compound (A) is 1 or more and 4 or less.

8. The carbon dioxide absorbent according to any one of claims 1 to 7, wherein the heterocyclic structure (X) comprises at least one selected from the group consisting of a 5-membered ring and a 6-membered ring.

9. The carbon dioxide absorbent according to any one of claims 1 to 8, wherein the amine compound (A) comprises at least one selected from the group consisting of 2,5-bisaminomethylfuran and derivatives thereof, 2,5-bis(aminomethyl)tetrahydrofuran and derivatives thereof, furfurylamine and derivatives thereof, tetrahydrofurfurylamine and derivatives thereof, 4-aminomethyltetrahydropyran and derivatives thereof, 4-(2-aminoethyl)morpholine and derivatives thereof, and 2-thiophenemethylamine and derivatives thereof.

10. The carbon dioxide absorbent according to any one of claims 1 to 9, wherein the carbon dioxide absorbent has a water content of 30% by mass or less.

11. The carbon dioxide absorbent according to any one of claims 1 to 10, wherein the carbon dioxide absorbent is used for directly absorbing carbon dioxide from the air.
